# EUROPÄISCHE PATENTSCHRIFT

(11) **EP 3 149 434 B1**
(45) Veröffentlichungstag und Bekanntmachung des Hinweises auf die Patenterteilung: **03.10.2018**
(21) Anmeldenummer: 15737972.8
(22) Anmeldetag: 22.05.2015
(51) Int. Cl.: G01N 33/18, G01N 25/00

(54) **ANALYSEGERÄT ZUR WASSER- UND ABWASSERANALYSE**
ANALYSIS DEVICE FOR ANALYZING WATER AND WASTEWATER
ANALYSEUR POUR L'ANALYSE DE L'EAU ET DES EAUX USÉES

(30) Priorität: 26.05.2014 DE 202014102443 U
(43) Veröffentlichungstag der Anmeldung: 05.04.2017
(73) Patentinhaber: LAR Process Analysers AG, 12057 Berlin (DE)
(72) Erfinder: ARTS, Werner, 10825 Berlin (DE); GENTHE, Wolfgang, 14059 Berlin (DE)
(74) Vertreter: Heinze, Ekkehard
(86) Internationale Anmeldenummer: PCT/DE2015/200321
(87) Internationale Veröffentlichungsnummer: WO 2015/180721

(56) Entgegenhaltungen:
- EP-A1- 2 466 304
- EP-A2- 2 221 610
- WO-A1-2012/044163
- CN-U- 203 492 280
- DE-A1- 2 908 169
- DE-A1- 19 546 952
- US-A- 3 560 156
- US-A- 3 560 156
- US-A- 3 842 679
- US-A- 5 824 886
- US-A- 5 824 886
- US-A- 5 841 884
- US-A1- 2007 254 374
- Skalar Analytical: "Formacs HT Total Organic Carbon Analyzer", , 16. Oktober 2012 (2012-10-16), XP054977854, Gefunden im Internet: URL:https://www.youtube.com/watch?v=Ok6nBa nIPVY [gefunden am 2017-11-03]
- Skalar: "Total Organic Carbon and Total Nitrogen analyzers SERIES Formacs SERIES | Total Organic Carbon and Total Nitrogen analyzers https", , 27. Januar 2013 (2013-01-27), XP055421675, Gefunden im Internet: URL:https://web.archive.org/web/2013012723 1717/http://www.skalar.com:80/analyzers/to tal-organic-carbon-toc-and-total-nitrogen- tn-analyzers [gefunden am 2017-11-03]
- Skalar Analytical: "Formacs HT Total Organic Carbon Analyzer", , 16 October 2012 (2012-10-16), XP054977854, Retrieved from the Internet: URL:https://www.youtube.com/watch?v=Ok6nBa nIPVY [retrieved on 2017-11-03]
- Skalar: "Total Organic Carbon and Total Nitrogen analyzers SERIES Formacs SERIES | Total Organic Carbon and Total Nitrogen analyzers https", , 27 January 2013 (2013-01-27), XP055421675, Retrieved from the Internet: URL:https://web.archive.org/web/2013012723 1717/http://www.skalar.com:80/analyzers/to tal-organic-carbon-toc-and-total-nitrogen- tn-analyzers [retrieved on 2017-11-03]

## Beschreibung

Die Erfindung betrifft ein Analysegerät, insbesondere zur Wasser- und Abwasseranalyse, mit einem Gerätegehäuse, welches Gerätekomponenten aufnimmt.

Derartige Analysegeräte sind typischerweise mit geschlossenen Gehäusen versehen, die für Wartungsarbeiten oder für nicht allzu häufig vorkommende Prüf- bzw. Einstellvorgänge an den Gerätekomponenten partiell demontiert werden müssen. Bei Geräten, die eine relativ häufige Prüfung, Einstellung oder Wartung bestimmter Gerätekomponenten erfordern, ist das Gehäuse mitunter auch schrankartig ausgeführt, also mit einer die Vorderfront einnehmenden Tür versehen, die zum Zwecke einer ersten Sichtkontrolle der Gerätekomponenten auch als Glastür ausgeführt sein kann.

Der Erfindung liegt die Aufgabe zu Grunde, ein hinsichtlich seiner praktischen Handhabung verbessertes Analysegerät der gattungsgemäßen Art anzugeben.

Diese Aufgabe wird nach einem ersten Aspekt der Erfindung gelöst durch ein Analysegerät mit den Merkmalen des Anspruchs 1. Zweckmäßige Fortbildungen des Erfindungsgedankens sind Gegenstand der abhängigen Ansprüche.

Die Erfindung schließt zunächst den Gedanken ein, an einer Gehäusefläche des Gerätegehäuses eine werkzeugfrei zu öffnende und zu verschließende Öffnung vorzusehen, die so konfiguriert ist, dass prüf- bzw. wartungsbedürftige Gerätekomponenten hierdurch nach außerhalb des Gerätes gebracht werden können. Gemäß einem zweiten Gedanken ist hierzu ein Komponentenschlitten oder -einsatz vorgesehen, der die relevanten Gerätekomponenten trägt und mit diesen zusammen derart konfiguriert und angeordnet ist, dass er durch die Öffnung mindestens teilweise nach außen transportiert werden kann. Unter einem Schlitten wird hierbei ein in einer Gleitführung verschiebliches Teil verstanden, während unter einem Einsatz ein auf andere Weise (etwa durch manuelles Herausheben oder auch durch Schwenken) durch die Öffnung transportierbares Teil verstanden wird.

Gemäß einer ersten Ausführung der Erfindung ist zum Verschließen der Öffnung eine an einer Gehäusekante angeschlagene Drehtür oder Klappe vorgesehen. Im Unterschied zu bekannten Analysegeräten ist eine solche Tür aber nicht als die gesamte Gerätefront einnehmendes Teil gestaltet. In einer alternativen Ausführung ist an der Vorderkante des Komponentenschlittens oder -einsatzes eine Verschlussplatte zum Verschließen der Öffnung bei eingezogenem Komponentenschlitten oder eingefügtem Komponenteneinsatz angebracht.

In einer Ausgestaltung beider Ausführungen ist die Drehtür oder Klappe oder Verschlussplatte zur Brmögüchung eines Luftaustauschs und/oder zur Wärmeabführung aus dem Gehäuseinneren perforiert.

Gemäß der Erfindung nimmt die Öffnung im Gerätegehäuse nur einen Teil der Gehäusefront, insbesondere zwischen 25 und 75 % und noch spezieller zwischen 40 und 60 %, ein, und der in Anpassung hieran konfigurierte Komponentenschlitten oder -einsatz nimmt einen entsprechenden Anteil des Volumens des Gerätegehäuses ein. Dies gewährleistet, dass am verbleibenden Teil der Gehäusefront Bedien- und/oder Anzeigeelemente angebracht sein können. In einer alternativen Ausgestaltung ist die Öffnung im Gerätegehäuse in einer Seitenfläche desselben vorgesehen, derart, dass die Gehäusefront im Wesentlichen vollständig für Bedien- und/oder Anzeigeelemente nutzbar ist.

Vorteile und Zweckmäßigkeiten der Erfindung ergeben sich im Übrigen aus der nachfolgenden kurzen Beschreibung eines Ausführungsbeispiels anhand der Figuren. Von diesen zeigen:
Fig. 1 eine schematische Darstellung wesentlicher Gerätekomponenten eines Analysegerätes gemäß einem Ausführungsbeispiel und
Fig. 2 eine perspektivische Darstellung jenes Analysegerätes.

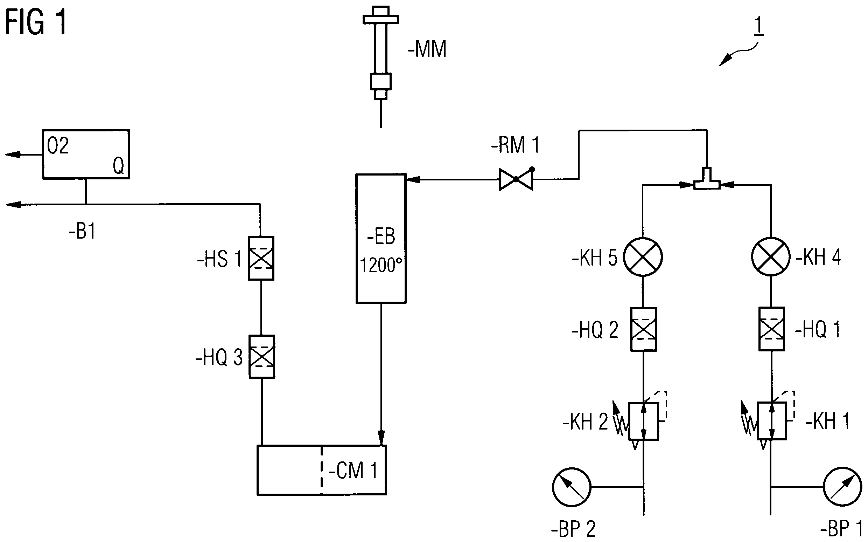

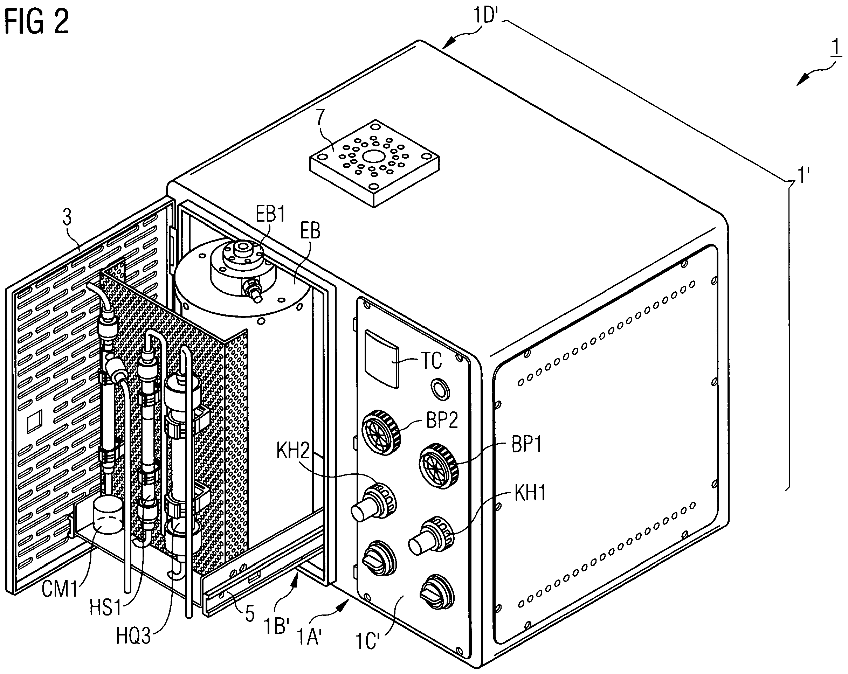

Fig. 1 und 2 zeigen schematisch wesentliche Teile eines Analysegerätes 1 zur Bestimmung des chemischen Sauerstoffbedarfs (CSB oder COD = chemical oxygen demand) von Wasser oder Abwasser. Der grundsätzliche Aufbau derartiger Geräte und deren Funktionsweise sind dem Fachmann bekannt und werden daher hier nicht näher beschrieben, zumal es hierauf zum Verständnis der vorliegenden Erfindung nicht ankommt.

Das Analysegerät 1 umfasst ein thermisches Reaktionsgefäß bzw. einen Ofen EB, in den mittels einer Injektionsspritze MM eine Wasserprobe eingespritzt werden kann und in dem diese Probe thermisch aufgeschlossen wird. Dem Ofen wird über ein Rückschlagventil RM1 ein Trägergasstrom zugeführt, welcher aus Luft und Stickstoff zusammengesetzt wird. Der Trägergasstrom wird mittels eines Luft-Druckreglers KH1, eines Stickstoff-Druckreglers KH2, eines Luft-Durchflussreglers KH4 und eines Stickstoff-Durcbfäussregters KH5 gesteuert und mittels eines ersten und zweiten Feinfilters HQ1, HQ2 eingangsseitig des Ofens gefiltert. Eingangsseitig des Ofens sind auch eine Luft-Druckanzeige BP1 und eine Stickstoff-Druckanzeige BP2 vorgesehen.

Ausgangsseitig des Ofens gelangt der Gasstrom zunächst in ein Kondensatgefäß CM1, und der nicht kondensierte Anteil durchläuft anschließend ein Quarzwollefilter HQ3 und eine Säurefalle HS1, bevor er zum Sauerstoffdetektor B1 gelangt, welcher schließlich einen (elektrischen) Messwert an (nicht dargestellte) weitere Gerätekomponenten zur Signalverarbeitung, -speicherung und -darstellung ausgibt.

Fig. 2 zeigt das Analysegerät 1 in perspektivischer Darstellung in einem Zustand, in dem das Gerätegehäuse 1' teilweise geöffnet sind und ein Teil der Gerätekomponenten aus dem Gehäuse herausgezogen ist. Das Gerätegehäuse hat im Wesentlichen die Gestalt eines quadratischen Prismas, und in der Gerätefront 1A' ist eine Öffnung 1B' vorgesehen, die durch eine an der linken Seitenkante der Gerätefront angeschlagene, perforierte Tür 3 zu verschließen ist.

Ein in seinen Abmessungen an die Öffnung 1B' angepasster Schlitten 5, auf dem der Ofen EB, das Kondensatgefäß CM1, das Quarzwollefüter HQ3 und die Säurefalle HS1 angeordnet sind, ist so weit aus dem Gehäuse herausziehbar, dass die genannten Komponenten frei zugänglich werden. Im zurückgeschobenen Zustand des Schlittens 5 wird das Gerätegehäuse 1' mit der Tür 3 verschlossen.

Auf der rechten Seite der Gerätefront 1A' befindet sich ein Bedienfeld 1C', auf dem mehrere Bedien- und Anzeigeelemente angeordnet sind, darunter eine Temperaturaszeige /-steuerung TC und die Einstellregler KH1 und KH2 für den Luft- bzw. Stickstoffdruck und die zugehörigen Anzeigeelemente BP1 und BP2. Auf der Geräteoberseite 1D' befindet sich ein Injektionsport 7, dessen Aufbau und Abmessungen an diejenigen der in Fig. 1 gezeigten Injektionsspritze MM angepasst sind und der im Geräteinneren mit einem entsprechenden Einspritzventil EB1 des Ofens EB kommuniziert, wenn der Ofen sich in seiner normalen Gebrauchslage Innerhalb des Gerätegehäuses befindet.

Die Ausführung der Erfindung ist nicht auf dieses Beispiel beschränkt, sondern ebenso in einer Vielzahl von Abwandlungen möglich, die im Rahmen der folgenden Ansprüche liegen.

## Patentansprüche

1. Analysegerät, insbesondere zur Wasser- und Abwasseranalyse, mit einem Gerätegehäuse, welches Gerätekomponenten aufnimmt und welches an der Gehäusefront eine werkzeugfrei öffen- und verschließbare Öffnung hat, und
einem Komponentenschlitten oder -einsatz, der einen Teil der Gerätekomponenten trägt und mit diesen zusammen derart konfiguriert und angeordnet ist, dass er durch die Öffnung mindestens teilweise nach außen transportiert werden kann, derart, dass die auf ihm angeordneten Gerätekomponenten leicht zugänglich werden,
wobei die Öffnung im Gerätegehäuse nur einen Teil der Gehäusefront, insbesondere zwischen 25 und 75 % und noch spezieller zwischen 40 und 60 %, einnimmt und der in Anpassung hieran konfigurierte Komponentenschlitten oder - einsatz einen entsprechenden Anteil des Volumens des Gerätegehäuses einnimmt, wobei am verbleibenden Teil der Gehäusefront Bedien- und/oder Anzeigeelemente vorgesehen sind; und wobei
auf der Geräteoberseite ein Injektionsport zum Einspritzen einer Flüssigkeit bei geschlossenem Gerätegehäuse vorgesehen ist, und mit einem im Analysegerät und auf dem Komponentenschlitten oder - einsatz untergebrachten Reaktionsgefäß zum thermischen Aufschluss des zu analysierenden Stoffes verbunden ist.

2. Analysegerät nach Anspruch 1, wobei zum Verschließen der Öffnung an der Gehäusefront eine an einer Gehäusekante angeschlagene Drehtür oder Klappe vorgesehen ist.

3. Analysegerät nach Anspruch 1, wobei an der Vorderkante des Komponentenschlittens oder -einsatzes eine Verschlussplatte zum Verschließen der Öffnung bei eingezogenem Komponentenschlitten oder eingefügtem Komponenteneinsatz angebracht ist.

4. Analysegerät nach Anspruch 2 oder 3, wobei die Drehtür oder Klappe oder Verschlussplatte zur Ermöglichung eines Luftaustauschs und/oder zur Wärmeabführung aus dem Gehäuseinneren perforiert ist.

5. Analysegerät nach einem der vorangehenden Ansprüche, wobei der Injektionsport auf der Geräteoberseite oberhalb des Komponentenschlittens oder -einsatzes angeordnet ist.

## Claims

1. An analysis device, in particular for analyzing water and wastewater, comprising
a device housing that holds device components and has an opening on the housing front, said opening being openable and closable without using any tools, and
a component carriage or component insert which supports part of the device components and is configured and arranged together with said device components in such a way that at least part of the carriage or insert can be conveyed out through the opening so that the device components placed thereon are easily accessible,
wherein the opening in the device housing occupies only a part of the housing front, in particular between 25 and 75%, and more specifically between 40 and 60%, and the component carriage or component insert configured to be adapted thereto occupies a corresponding proportion of the device housing's volume, operating and/or display elements being provided on the remaining part of the housing front; and wherein on an upper device surface an injection port is provided for injecting a liquid when the device housing is closed, which port is connected with a reaction vessel mounted on the component carriage or component insert, for thermally decomposing the substance to be analyzed.

2. The analysis device according to claim 1, wherein a pivot door or flap hinged to a housing edge on the housing front is provided for closing the opening.

3. The analysis device according to claim 1, wherein a closing plate is provided at the front edge of the component carriage or component insert for closing the opening when the component carriage is retracted or the component insert is inserted.

4. The analysis device according to claim 2 or 3, wherein the pivot door or flap or closing plate is perforated for enabling air to be exchanged and/or for dissipating heat from the housing's interior.

5. The analysis device according to one of the preceding claims, wherein the injection port is disposed on the upper side of the device and above the component carriage or component insert

## Revendications

1. Appareil d'analyse, en particulier pour l'analyse de l'eau et des eaux usées, comportant un boîtier d'appareil qui renferme des composants de l'appareil et qui présente, sur l'avant du boîtier, une ouverture ouvrable et refermable sans outils, et
un chariot ou un insert de composants qui porte une partie des composants de l'appareil et est configuré et agencé conjointement avec ceux-ci de sorte qu'il peut être au moins en partie transporté vers l'extérieur à travers l'ouverture, et de sorte que les composants de l'appareil agencé sur lui sont facilement accessibles,
dans lequel l'ouverture dans le boîtier de l'appareil n'occupe qu'une partie de l'avant du boîtier, en particulier entre 25 et 75 %, et plus spécialement entre 40 et 60 %, et le chariot ou l'insert de composants configuré de manière adaptée en conséquence occupe une part correspondante du volume du boîtier de l'appareil, des éléments de commande et/ou d'affichage étant prévus sur la partie restante de l'avant de l'appareil ; et dans lequel
il est prévu sur la face supérieure de l'appareil un orifice d'injection pour injecter un liquide lorsque le boîtier de l'appareil est fermé, et qui est relié avec un récipient de réaction installé dans l'appareil d'analyse et sur le chariot ou l'insert de composants à des fins d'évaluation thermique du matériau qu'il s'agit d'analyser.

2. Appareil d'analyse selon la revendication 1, dans lequel il est prévu une porte rotative ou un clapet articulé(e) sur une arête du boîtier pour fermer l'ouverture à l'avant du boîtier.

3. Appareil d'analyse selon la revendication 1, dans lequel une plaque de fermeture est fixée sur l'arête avant du chariot ou de l'insert de composants pour fermer l'ouverture lorsque le chariot de composants est entré ou que l'insert de composants est introduit.

4. Appareil d'analyse selon la revendication 2 ou 3, dans lequel la porte rotative ou le clapet ou la plaque de fermeture est perforé(e) pour permettre un échange d'air et/ou une évacuation de chaleur avec l'intérieur du boîtier.

5. Appareil d'analyse selon l'une quelconque des revendications précédentes, dans lequel l'orifice d'injection est agencée sur la face supérieure de l'appareil au-dessus du chariot ou de l'insert de composants.
